# EUROPEAN PATENT APPLICATION

(11) **EP 2 287 788 A1**
(43) Date of publication of application: **23.02.2011**
(21) Application number: 10008718.8
(22) Date of filing: 20.08.2010
(51) Int. Cl.: G06Q 10/00, G06K 19/067

(54) **Method, mobile terminal, transponder device and system for monitoring an object with respect to the mobile terminal**

(30) Priority: 20.08.2009 EP 09010704; 20.08.2009 US 235446 P
(71) Applicant: Deutsche Telekom AG, 53113 Bonn (DE)
(72) Inventor: Hillebrand, Rainer, 48268 Greven (DE); Effelsberg, Volker, 53225 Bonn (DE)
(74) Representative: Schwöbel, Karl T.

(57) **Abstract**

The invention relates to a method, a mobile terminal, a transponder device, a system, a program comprising a computer readable program code and a computer program product for monitoring an object, the mobile terminal having a short range radio interface, the object comprising the transponder device or being attached to a transponder device, the transponder device being operable to communicate with the mobile terminal via the short range radio interface, the method comprising the step of the mobile terminal receiving a radio signal emitted by the transponder device indicating that the object is present within the coverage area of the short range radio interface of the mobile terminal.

## Description

### BACKGROUND

The present invention relates to a method, a mobile terminal, a transponder device and a system that allows the monitoring of an object, especially the presence of the object, with respect to the mobile terminal.

Therapy to patients suffering especially from chronic diseases usually takes a long period of time. Such persons require permanent medical treatment, especially by means of drugs or medicines to be administered in regular intervals. Severe consequences can occur if the administration of such drugs or medicines is not performed properly. So far, a patient needs to remember to always take along such an essential drug or medicine in the event he or she, e.g., leaves his or her home or another location.

In case a patient unintentionally forgets to take along such a drug or medicine resulting in a medical emergency, emergency calling systems are known that aim at providing help to such patients. However, such systems usually require reaching a fixed line telephone or an associated device.

Presently, there are many different types of transponder systems known, examples of which include contactless smart cards or electronic labels. A characteristic hitherto common to so-called passive transponders or transponder devices of various designs is that they first receive energy from an emitting terminal (also known as the base station, reader station or reader), with which they then communicate. The energy is supplied by the terminal in the form of an alternating electromagnetic field. With an antenna or a coil, the transponder absorbs a portion of the energy of the electromagnetic field and thereby supplies its electronic circuits. The frequency ranges of the alternating field are generally in the order of about 100 KHz to a few GHz.

Furthermore, so-called active transponders or transponder devices are known, e.g. the so-called RuBee or ZigBee transponders. Such an active transponder has a battery from which it receives the electrical operating energy required for transmission. In this case, an activation radio frequency signal (received by the transponder) can be used for addressing purposes, to wake up and thus to activate the transponder. RuBee denotes a bi-directional protocol based on the Institute of Electrical and Electronics Engineers (IEEE) 1902.1 standard and operates at frequencies below 450 kHz (optimally at a low frequency band of 132 kHz). RuBee has a transmission range of up 15 m to 30 m in radius and supports a data rate of up to 100 Kbps, and RuBee waves can pass through water and ground. ZigBee denotes communication protocols based on the IEEE 802.15.4 standard, and operates in the Industrial, Scientific and Medical (ISM) radio bands of 868 MHz, 915 MHz, and 2.4 GHz. ZigBee has a transmission range of 10 to 20 m in radius and supports a data rate of 20 to 250 Kbps.

Generally, such transponders or transponder devices operate in the radio frequency range from 3 to 30 MHz, although appropriate different wavelengths, such as low frequency from 30 kHz to 300 kHz, ultra-high frequency from 300 MHz to 3 GHz or microwave frequencies above 3 GHz may also be used depending on the desired range or coverage area.

### SUMMARY

The present invention aims at preventing such above described medical emergency situations by means of alerting a patient of the fact that he or she is moving beyond a certain distance from his or her essential drugs or medicines. More generally, the present invention aims at alerting a person of the fact that he or she is moving beyond a certain distance from a special object essential to his or her well-being.

It is therefore an object of the present invention to provide a method, a mobile terminal, a transponder device and a system that allows the monitoring of an object, especially the presence of the object, with respect to the mobile terminal such that a user can be informed about the fact that he or she is moving beyond a certain distance from such an object.

The problem is solved by means of a system for monitoring an object with respect to a mobile terminal, the system comprising the mobile terminal and a transponder device, the mobile terminal comprising a short range radio interface, the mobile terminal being operable to communicate with the transponder device via the short range radio interface, wherein the object comprises the transponder device or is attached to the transponder device, wherein the mobile terminal is operable for receiving a radio signal emitted by the transponder device indicating that the object is present within the coverage area of the short range radio interface of the mobile terminal, wherein the mobile terminal has a further radio interface, the further radio interface being one of a wireless wide area network interface or a wireless local area network interface, wherein the transponder device comprises an acceleration sensor operable to provide an acceleration signal, wherein in case that the radio signal is not received within a predetermined time interval by the mobile terminal, the mobile terminal is provided such that an alert signal is output by the mobile terminal, wherein the radio signal emitted by the transponder device is emitted repeatedly within a further predetermined time interval, wherein in case that the object is detected as not being present within the coverage area of the short range radio interface of the mobile terminal, a communication of the mobile terminal with another party of a communications network and a transmission of the alert signal is provided, wherein the emission of the radio signal by the transponder device depends on the acceleration signal.

According to the present invention, it is thereby advantageously possible that - by means of the mobile terminal receiving the radio signal from the transponder device - the mobile terminal receives the information that the transponder device is located within a comparably short distance from the mobile terminal. This means that if, e.g., the transponder device is either integrated in, e.g., a packaging of an essential drug or medicine or attached to an essential drug or medicine, it is possible to alert a user of the fact that at least no radio signal has been received by the mobile terminal for a certain time interval. If the assumption is met that the mobile terminal is generally carried by the user relatively closely to his or her body or that the mobile terminal is virtually always taken along by the user (e.g. in a bag or the like), the failure (of the mobile terminal) to receive the radio signal from the transponder device (within a predetermined time interval) is a strong indicator that the distance between the transponder device (and hence the essential drug or medicine or other object) on the one hand and the mobile terminal (and hence the user) on the other hand exceeds the range of the short range radio interface which can be of the order of magnitude of a few meters until a few hundreds of meters, being preferably between 5 meters and 100 meters and more preferably between 10 meters and 30 meters. If the user is alerted of such a situation (i.e. that the essential drug or medicine is further away than the range or the short range radio interface), it is usually possible without too much effort to get the essential drug or medicine or to take along the essential drug or medicine because the distance between the user and the mobile terminal is comparably low. The alert is usually done by means of an alert signal generated by the mobile terminal. The alert signal can be any acoustical and/or optical and/or mechanical (vibrational) signal of the mobile terminal that can be detected by the user, e.g. a beeping sound and/or a melody and/or an illuminated LED and/or a flash light and/or a vibrational signal. According to the present invention, the mobile terminal therefore comprises a corresponding output device, i.e. an acoustic output device like a loudspeaker, and/or an optical output device like an LED, and/or a mechanical output device like a vibration generator.

According to the present invention, the predetermined time interval is a time interval of, e.g., several seconds to several minutes or several hours. The definition of the predetermined time interval might depend, e.g., on the individual health situation of the user and/or on the individual administration scheme of the drug or the medicine.

According to the present invention, the radio signal emitted by the transponder device is emitted repeatedly within a further predetermined time interval, e.g. every several seconds or every several minutes or every several hours. The definition of the further predetermined time interval might also depend, e.g., on the individual health situation of the user and/or on the individual administration scheme of the drug or the medicine.

By means of adjusting the preferred time interval and the further preferred time interval, it is advantageously possible to provide for an effective alerting of the user by the mobile terminal in case the radio signal is not received by the mobile terminal, i.e. in case the object is detected as not being present within the coverage area of the short range radio interface of the mobile terminal. For example, it is possible that the predetermined time interval is comparable to the further predetermined time interval, which means that every radio signal emitted by the transponder device has to be received by the mobile terminal, otherwise the alert signal is generated by the mobile terminal. Alternatively, it is also possible that the predetermined time interval is longer (e.g. twice as long or thrice as long) than the further predetermined time interval, which means that at least one occurrence (or two subsequent occurrences) of the radio signal emitted by the transponder device and not received by the mobile terminal is/are tolerated without an alert signal being generated. The first alternative provides a higher level of security of (quickly) detecting a comparably high distance between the transponder device and the mobile terminal and the second alternative provides a lower probability of false positive alert signals.

It is also possible according to the present invention that the alert signal varies dependent on how many occurrences of the radio signal have not been received by the mobile terminal. This means that a first predetermined time interval and a second predetermined time interval are defined and the mobile terminal generates a first alert signal if the radio signal is not received within a first predetermined time interval by the mobile terminal and that the mobile terminal generates a second alert signal if the radio signal is not received within a second predetermined time interval by the mobile terminal. For example, it is possible according to the present invention that only an optical alert signal (as the first alert signal) is generated in case the radio signal has not been received within the first predetermined time interval (corresponding, e.g., to the further preferred time interval, i.e. the emission interval of the radio signal emitted by the transducer device) and that an optical and an acoustic alert signal (as the second alert signal) is generated in case the radio signal has not been received within the second predetermined time interval (corresponding, e.g., to twice the further preferred time interval, i.e. twice the emission interval of the radio signal emitted by the transducer device).

According to the present invention, the mobile terminal has a further radio interface, the further radio interface being one of a wireless wide area network interface or a wireless local area network interface, wherein the method further comprises the step of the mobile terminal communicating with another party of a communications network and transmitting the alert signal in case the object is detected as not being present within the coverage area of the short range radio interface of the mobile terminal.

In this embodiment of the present invention according to which the mobile terminal comprises the further radio interface, it is advantageously possible not only to transmit the alert signal to the user carrying the mobile terminal (i.e. by means of the output device of the mobile terminal) but (additionally and/or alternatively) transmitting the alert signal to another party or location, e.g., by means of a communication of the mobile terminal via the further radio interface with a base station.

It is thereby advantageously possible that, e.g., the generation of the alert signal to the user of the mobile terminal (i.e. any acoustical and/or optical and/or mechanical (vibrational) signal of the mobile terminal that can be detected by the user) corresponds to the first alert signal and that the mobile terminal communicating with another party of a communications network (and transmitting the alert signal in case the object is detected as not being present within the coverage area of the short range radio interface of the mobile terminal) corresponds to the second alert signal.

According to the present invention, the other party of the communications network might be, e.g., a neighbor of the user and/or an emergency center. The other party can be alerted automatically, i.e. the information that the user of the inventive system is out of reach of essential drugs or medicines. Furthermore, it is possible that additional information is transmitted to the other party such as:
- an information regarding the geographical location of the user of the inventive system, and/or
- an information about the name or the nature of the essential drug or medicine or another information about the object to be monitored. Preferably, the further steps in handling the alert signal depend on the nature of the object to be monitored, e.g. on the nature of the drug or medicine.

Furthermore, it is possible according to the present invention that the communication between the mobile terminal and the other party is performed by means of a voice communication link (voice call) and/or by means of a data communication link, e.g. by means of an SMS (Short Message Service) service. It is furthermore preferred that the further radio interface does not require a specified fixed line telecommunication equipment such that the inventive system can be used wherever the further radio interface is available.

According to the present invention, it is preferred that the transponder device comprises an acceleration sensor operable to provide an acceleration signal, wherein the emission of the radio signal by the transponder device depends on the acceleration signal.

It is thereby advantageously possible to perform the method in an efficient way, especially in view to reduce the consumption of energy within the transponder device. In dependency of the acceleration signal, it is possible that the transponder device emits the radio signal such that the information is transmitted to the mobile terminal that no acceleration (of the transponder device) has occurred for a certain period of time. According to a preferred embodiment of the present invention, this information can be used to reduce the number of transmissions of the radio signal (during a certain period of time) from the transponder device to the mobile terminal, thus reducing the consumption of energy in the transponder device and possibly enhancing the life time of the battery (or other source of energy) of the transponder device.

According to the present invention, it is preferred that the emission of a further radio signal by the mobile terminal is provided, the radio signal emitted by the transponder device being received by the mobile terminal in response of the further radio signal.

In the case of the transponder device being an active transponder device, it is also possible that the further radio signal serves as a paging signal. This means that the radio signal of the transponder device is emitted in response to the further radio signal of the mobile terminal. In this case it is possible to provide a higher level of communication security (and hence a higher reliability of the inventive method) as it is possible, e.g., that the mobile terminal sends the further radio signal in case the mobile terminal has missed an expected radio signal (to be sent by the transponder device). In this situation a verification procedure (i.e. prompting the transponder device to emit the radio signal immediately once more) can lead to a quicker determination whether the transponder device (and hence the object) has been moved out of the range of the short range radio interface of the mobile terminal (and hence a quicker possibility to alert the user).

According to the present invention, it is furthermore preferred that the mobile terminal comprises a further acceleration sensor operable to provide a further acceleration signal, wherein the emission of the further radio signal by the mobile terminal depends on the further acceleration signal.

It is thereby advantageously possible to provide an efficient inventive system perform or to perform the inventive method in an efficient way, especially in view to reduce the consumption of energy within the transponder device and within the mobile terminal. In dependency of the further acceleration signal, it is possible that the mobile terminal emits the further radio signal such that the information is transmitted to the transponder device that no acceleration (of the mobile terminal) has occurred for a certain period of time. According to a preferred embodiment of the present invention, this information can be used to reduce the number transmissions of the radio signal (during a certain period of time) from the transponder device to the mobile terminal, thus reducing the consumption of energy in the transponder device and possibly enhancing the life time of the battery (or other source of energy) of the transponder device. For example, it is possible that both the mobile terminal and the transponder device do not emit the radio signal and the further radio signal (for a comparably long time) provided that both the acceleration signal and the further acceleration signal suggests that no (measurable) acceleration has occurred to the mobile terminal and to the transponder device.

It is furthermore preferred according to the present invention that all functions of the mobile terminal that are not required for the communication via the short range radio interface can be switched off, either actively or in dependency of the further acceleration signal. Thereby, the energy consumption of the mobile terminal can be reduced to a minimum.

The present invention also relates to a method for monitoring an object with respect to a mobile terminal, the mobile terminal having a short range radio interface, the object comprising a transponder device or being attached to a transponder device, the transponder device being operable to communicate with the mobile terminal via the short range radio interface, wherein the mobile terminal has a further radio interface, the further radio interface being one of a wireless wide area network interface or a wireless local area network interface, wherein the transponder device comprises an acceleration sensor operable to provide an acceleration signal, the method comprising the steps of
-- the mobile terminal receiving a radio signal emitted by the transponder device indicating that the object is present within the coverage area of the short range radio interface of the mobile terminal,
-- the mobile terminal outputting an alert signal if the radio signal is not received within a predetermined time interval by the mobile terminal,
-- the radio signal emitted by the transponder device being emitted repeatedly within a further predetermined time interval,
-- the mobile terminal communicating with another party of a communications network and transmitting the alert signal in case the object is detected as not being present within the coverage area of the short range radio interface of the mobile terminal,
-- the transponder device emitting the radio signal dependent on the acceleration signal.

According to the present invention, it is thereby advantageously possible that - by means of the mobile terminal receiving the radio signal from the transponder device - the mobile terminal receives the information that the transponder device is located within a comparably short distance from the mobile terminal.

According to the present invention, it is furthermore preferred that the emission of a further radio signal by the mobile terminal is provided, wherein the method comprises the step of the mobile terminal emitting the further radio signal, the radio signal emitted by the transponder device being received by the mobile terminal in response of the further radio signal.

Furthermore, it is preferred according to the present invention that the mobile terminal comprises a further acceleration sensor operable to provide a further acceleration signal, and wherein the method comprises the further step of the mobile terminal emitting the further radio signal dependent on the further acceleration signal.

According to a further embodiment of the present invention, it is preferred that the method further comprises the step of the mobile terminal identifying the transponder device, the step of the mobile terminal identifying the transponder device being performed prior to the reception of the radio signal by the mobile terminal. The identification of the transponder device is possible to perform, e.g., by means of inputting an identification number into the mobile terminal (for example by means of an input means like a keyboard or a touch screen or the like) and/or by means of recognizing a bar code (for example by means of a scanner or by means of an image capturing device like a camera or the like).

If more than one transponder devices exist in the environment of mobile terminal which are unknown to the mobile terminal (i.e. for which no identifications procedure has been performed) and one transponder device of such a plurality of transponder devices is to be identified by the mobile terminal, one strategy consists in reducing the power of the further radio signal and/or the sensitivity of for the reception of the radio signals emitted by the transponder devices such that only one transponder device (of the plurality of transponder devices) is reached by the mobile terminal or is seen by the mobile terminal.

According to the present invention, it is possible that the step of identifying the transponder device also encompasses the definition of the object to be monitored by the inventive method. In case the transponder device is attached to the object (i.e. the object like a drug packaging or the like is not connected to the transponder device (e.g. during manufacture of the object) in a permanent fashion), the nature of the object is unknown to the mobile terminal. Therefore, the definition of the object to be monitored comprises, e.g., to input a product code of the object to be monitored in the mobile terminal. The definition of the object can be performed, e.g., by means of inputting an identification number into the mobile terminal (for example by means of an input means like a keyboard or a touch screen or the like) and/or by means of recognizing a bar code (for example by means of a scanner or by means of an image capturing device like a camera or the like).

According to the present invention, it is possible that the bar code information is transmitted via the further radio interface such that the mobile terminal receives the relevant information regarding the transponder device and/or regarding the product.

Dependent on the nature of the object to be monitored, it is possible according to the present invention to store different alerting schemes corresponding to different risk levels or risk classes such that, e.g., the alert signal related to less essential drug or medicine (or other object) is handled differently to the alert signal indicating that a more essential drug or medicine (or other object) is out of reach of the user.

The invention also relates to a transponder device for use with the inventive method, the transponder device being operable to communicate with a mobile terminal via a short range radio interface, the mobile terminal comprising the short range radio interface, wherein an object comprises the transponder device or is attached to the transponder device, wherein the transponder device is operable for transmitting a radio signal to the mobile terminal indicating that the object is present within the coverage area of the short range radio interface of the mobile terminal, wherein the transponder device comprises an acceleration sensor, wherein the acceleration sensor is operable to provide an acceleration signal, and wherein the transponder device emits the radio signal dependent on the acceleration signal.

The invention further relates to a mobile terminal for use with the inventive method, the mobile terminal comprising a short range radio interface, the mobile terminal being operable to communicate with a transponder device via the short range radio interface, wherein an object comprises the transponder device or is attached to the transponder device, wherein the mobile terminal is operable for receiving a radio signal emitted by the transponder device indicating that the object is present within the coverage area of the short range radio interface of the mobile terminal.

Preferably, the mobile terminal has a further radio interface, the further radio interface being one of a wireless wide area network interface or a wireless local area network interface, wherein the method further comprises the step of the mobile terminal communicating with another party of a communications network and transmitting the alert signal in case the object is detected as not being present within the coverage area of the short range radio interface of the mobile terminal.

The invention further relates to program comprising a computer readable program code and to a computer program product comprising a computer readable program code for controlling a mobile terminal, the mobile terminal comprising a short range radio interface, the mobile terminal being operable to communicate with a transponder device via the short range radio interface, wherein an object comprises the transponder device or is attached to the transponder device, wherein the mobile terminal is operable for receiving a radio signal emitted by the transponder device indicating that the object is present within the coverage area of the short range radio interface of the mobile terminal.

These and other characteristics, features and advantages of the present invention will become apparent from the following detailed description, taken in conjunction with the accompanying drawings, which illustrate, by way of example, the principles of the invention. The description is given for the sake of example only, without limiting the scope of the invention. The reference figures quoted below refer to the attached drawings.

### BRIEF DESCRIPTION OF THE DRAWINGS

Figure 1 schematically shows an example of a system for monitoring an object with respect to a mobile terminal.

Figures 2 and 3 schematically show two different modes of communication between a transponder device and the mobile terminal according to the present invention.

Figures 4 and 5 schematically show two examples of the transponder device according to the present invention.

### DETAILED DESCRIPTION

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated.

Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described of illustrated herein.

According to the present invention, an object can be monitored with respect to a mobile terminal.

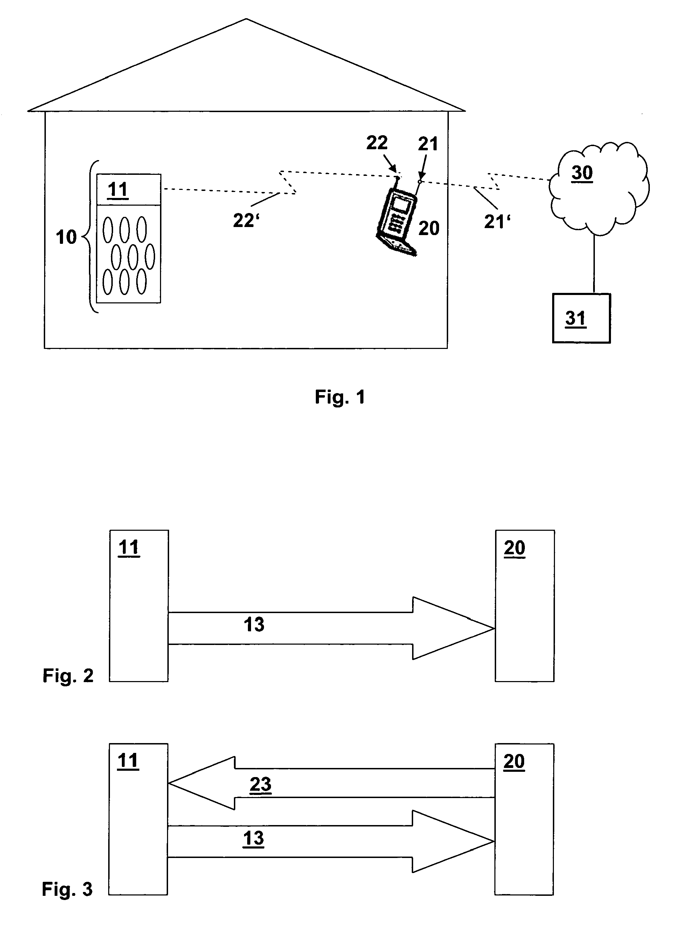

Figure 1 schematically shows an example of a system for monitoring an object 10 with respect to the mobile terminal 20. The object 10 is either attached to a transponder device 11 or the object 10 is manufactured with the transponder device 11 integrated in the object 10 so that the object 10 comprises the transponder device 11. The mobile terminal 20 comprises a short range radio interface 22 and the transponder device 11 is operable to communicate with the mobile terminal 20 via the short range radio interface 22. The short range radio interface 22 is preferably a radio interface suitable to be used between the mobile terminal 20 and the transponder device 11, the mobile terminal 22 acting (by means of the short range radio interface 22) as reader station to the transponder device 11. Examples of the short range radio interface and short range communication link 22' established between the mobile terminal 20 and the transponder device 11 include:
- a RFID short range radio interface,
- a NFC (Near Field Communication) short range radio interface,
- a RuBee short range radio interface.

According to a very preferred embodiment of the mobile terminal 20, the mobile terminal 20 comprises also a further radio interface 21 operable to connect the mobile terminal 20 to another party 31 of a communications network 30. The further radio interface 21 is preferably one of a wireless wide area network interface or a wireless local area network interface. Examples of the further radio interface 21 and further communication link 21' established between the mobile terminal 20 and the communications network 30 include:
- a cellular mobile communications network interface,
- a WLAN (Wireless Local Area Network) interface.

According to the present invention, the mobile terminal 20 comprises a memory device (not shown) such that data related to the transponder device 11 monitored or related to a plurality of transponder devices 11 monitored are stored in the memory device. In case of an emergency situation necessitating a communication via the further radio interface 21, such data related to the transponder device 11 can be transmitted to the communications network 30 and/or to the other party 31. Such data can, e.g., comprise monitoring data such as:
- a time stamp of the last contact to the transponder device 11 and/or
- the geographical coordinates of the mobile terminal at present and/or at the time of the last contact to the transponder device 11.

Preferably, the mobile terminal stores information about any established communication link to the other party 31 and/or about any transmitted data.

Figures 2 and 3 schematically show two different modes of communication between the transponder device 11 and the mobile terminal 20 according to the present invention.

Figure 2 shows the case of the transponder device 11 being an active transponder. The transponder device 11 emits a radio signal 13 which is received by the mobile terminal 20. The radio signal 13 is preferably emitted in regular time intervals (also called further predetermined time interval in the context of the present invention). The transponder device 11 does not need to be incited by the mobile terminal 20 to emit the radio signal 13. Of course, it is possible according to the present invention that the transponder device 11 is incited by the mobile terminal 20 to emit the radio signal 13 (not shown in Figure 2).

Figure 3 shows the case of the transponder device 11 being a passive transponder. The transponder device 11 emits the radio signal 13 in response of a further radio signal 23 emitted by the mobile terminal 20 to the transponder device 11. The transponder device 11 uses the energy of the further radio signal 23 to generate the radio signal 13 which is received by the mobile terminal 20. The further radio signal 23 and thus the radio signal 13 is preferably emitted in regular time intervals (also called further predetermined time interval in the context of the present invention). By means of the further radio signal 23, the transponder device 11 is always incited by the mobile terminal 20 to emit the radio signal 13.

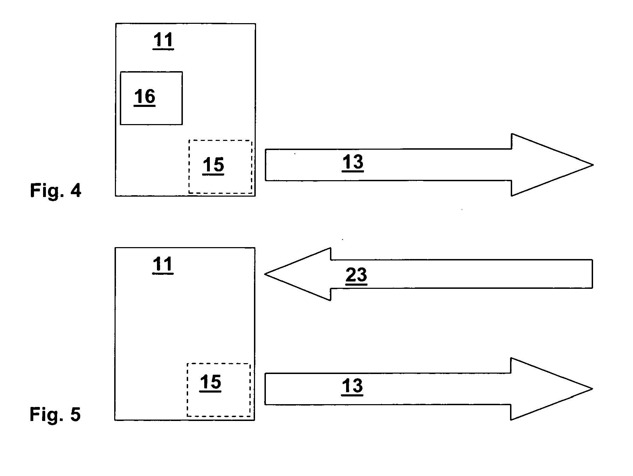

Figures 4 and 5 schematically show two examples of the transponder device 11 according to the present invention.

Figure 4 shows the case of the transponder device 11 being an active transponder. The transponder device 11 comprises a source of energy 16, e.g. a battery. The transponder device 11 emits the radio signal 13 which is received by the mobile terminal 20. According to a preferred embodiment of the present invention, the transponder device 11 comprises an acceleration sensor 15.

Figure 5 shows the case of the transponder device 11 being a passive transponder. The transponder device 11 does not comprise a source of energy but receives the further radio signal 23 and uses the energy of the further radio signal 23 to generate the radio signal 13 which is received by the mobile terminal 20. According to a preferred embodiment of the present invention, the transponder device 11 comprises an acceleration sensor 15.

## Claims

1. A system for monitoring an object (10) with respect to a mobile terminal (20), the system comprising the mobile terminal (20) and a transponder device (12), the mobile terminal (20) comprising a short range radio interface (22), the mobile terminal (20) being operable to communicate with the transponder device (12) via the short range radio interface (22), wherein the object (10) comprises the transponder device (12) or is attached to the transponder device (12), wherein the mobile terminal (20) is operable for receiving a radio signal (13) emitted by the transponder device (12) indicating that the object (10) is present within the coverage area of the short range radio interface (22) of the mobile terminal (20), wherein the mobile terminal (20) has a further radio interface (21), the further radio interface (21) being one of a wireless wide area network interface or a wireless local area network interface, wherein the transponder device (12) comprises an acceleration sensor (15) operable to provide an acceleration signal, wherein in case that the radio signal (13) is not received within a predetermined time interval by the mobile terminal (20), the mobile terminal (20) is provided such that an alert signal is output by the mobile terminal (20), wherein the radio signal (13) emitted by the transponder device (12) is emitted repeatedly within a further predetermined time interval, wherein in case that the object (10) is detected as not being present within the coverage area of the short range radio interface (22) of the mobile terminal (20), a communication of the mobile terminal (20) with another party (31) of a communications network (30) and a transmission of the alert signal is provided, wherein the emission of the radio signal (13) by the transponder device (12) depends on the acceleration signal.

2. System according to claim 1, wherein the emission of a further radio signal (23) by the mobile terminal (20) is provided, the radio signal (13) emitted by the transponder device (12) being received by the mobile terminal (20) in response of the further radio signal (23).

3. System according to claim 2, wherein the mobile terminal (20) comprises a further acceleration sensor operable to provide a further acceleration signal, wherein the emission of the further radio signal (23) by the mobile terminal (20) depends on the further acceleration signal.

4. A method for monitoring an object (10) with respect to a mobile terminal (20), the mobile terminal (20) having a short range radio interface (22), the object (10) comprising a transponder device (12) or being attached to a transponder device (12), the transponder device (12) being operable to communicate with the mobile terminal (20) via the short range radio interface (22), wherein the mobile terminal (20) has a further radio interface (21), the further radio interface (21) being one of a wireless wide area network interface or a wireless local area network interface, wherein the transponder device (12) comprises an acceleration sensor (15) operable to provide an acceleration signal, the method comprising the steps of
-- the mobile terminal (20) receiving a radio signal (13) emitted by the transponder device (12) indicating that the object (10) is present within the coverage area of the short range radio interface (22) of the mobile terminal (20),
-- the mobile terminal (20) outputting an alert signal if the radio signal (13) is not received within a predetermined time interval by the mobile terminal (20),
-- the radio signal (13) emitted by the transponder device (12) being emitted repeatedly within a further predetermined time interval,
-- the mobile terminal (20) communicating with another party (31) of a communications network (30) and transmitting the alert signal in case the object (10) is detected as not being present within the coverage area of the short range radio interface (22) of the mobile terminal (20),
-- the transponder device (12) emitting the radio signal (13) dependent on the acceleration signal.

5. Method according to claim 4, wherein the emission of a further radio signal (23) by the mobile terminal (20) is provided, wherein the method comprises the step of the mobile terminal (20) emitting the further radio signal (23), the radio signal emitted by the transponder device (12) being received by the mobile terminal (20) in response of the further radio signal (23).

6. The method according to claim 4 or 5, wherein the mobile terminal (20) comprises a further acceleration sensor operable to provide a further acceleration signal (23), and wherein the method comprises the further step of the mobile terminal (20) emitting the further radio signal (23) dependent on the further acceleration signal.

7. The method according to one of claims 4 to 6, wherein the method further comprises the step of the mobile terminal (20) identifying the transponder device (11), the step of the mobile terminal (20) identifying the transponder device (11) being performed prior to the reception of the radio signal by the mobile terminal (20).

8. The method according to one of claims 4 to 7, wherein the object (10) is a drug or medicine packaging or a medicine container.

9. Transponder device (12) for use with a method according to claim 4, the transponder device (12) being operable to communicate with a mobile terminal (20) via a short range radio interface (22), the mobile terminal (20) comprising the short range radio interface (22), wherein an object (10) comprises the transponder device (12) or is attached to the transponder device (12), wherein the transponder device (12) is operable for transmitting a radio signal (13) to the mobile terminal (20) indicating that the object (10) is present within the coverage area of the short range radio interface (22) of the mobile terminal (20), wherein the transponder device (12) comprises an acceleration sensor (15), wherein the acceleration sensor (15) is operable to provide an acceleration signal, and wherein the transponder device (12) emits the radio signal (13) dependent on the acceleration signal.

10. Transponder device (12) according to claim 9, wherein the transponder device (12) is an active transponder device (12).

11. Mobile terminal (20) for use with a method according to claim 4, the mobile terminal (20) comprising a short range radio interface (22), the mobile terminal (20) being operable to communicate with a transponder device (12) via the short range radio interface (22), wherein an object (10) comprises the transponder device (12) or is attached to the transponder device (12), wherein the mobile terminal (20) is operable for receiving a radio signal (13) emitted by the transponder device (12) indicating that the object (10) is present within the coverage area of the short range radio interface (22) of the mobile terminal (20).

12. Mobile terminal according to claim 11, wherein the mobile terminal (20) has a further radio interface (21), the further radio interface (21) being one of a wireless wide area network interface or a wireless local area network interface, wherein the method further comprises the step of the mobile terminal (20) communicating with another party (31) of a communications network (30) and transmitting the alert signal in case the object (10) is detected as not being present within the coverage area of the short range radio interface (22) of the mobile terminal (20).

13. Program comprising a computer readable program code for controlling a mobile terminal (20) according to one of claims 11 or 12, the mobile terminal (20) comprising a short range radio interface (22), the mobile terminal (20) being operable to communicate with a transponder device (12) via the short range radio interface (22), wherein an object (10) comprises the transponder device (12) or is attached to the transponder device (12), wherein the mobile terminal (20) is operable for receiving a radio signal (13) emitted by the transponder device (12) indicating that the object (10) is present within the coverage area of the short range radio interface (22) of the mobile terminal (20).

14. Computer program product comprising a computer readable program code according to claim 13 for controlling a mobile terminal (20).
